# EUROPEAN PATENT SPECIFICATION

(11) **EP 2 174 675 B1**
(45) Date of publication and mention of the grant of the patent: **15.01.2014**
(21) Application number: 09012950.3
(22) Date of filing: 13.10.2009
(51) Int. Cl.: A61F 2/00, A61F 2/06, A61L 27/18, A61L 27/50, A61L 29/16

(54) **Textile implant of sheath-core construction and method of forming it**
Textilimplantat von Hüllen-Kernkonstruktionen und Herstellungsverfahren dafür
Implant textile ayant une gaine et son procédé de formation

(30) Priority: 13.10.2008 DE 102008052837
(43) Date of publication of application: 14.04.2010
(73) Proprietor: Aesculap AG, 78532 Tuttlingen/Donau (DE); HeiQ Materials AG, 5330 Bad Zurzach (CH)
(72) Inventor: Odermatt, Erich, Dr., 8200 Schaffhausen (CH); Berndt, Ingo, Dr., 78532 Tuttlingen (DE); Centonze, Carlo, Riccardo, 5466 Kaiserstuhl (CH); Height, Murray, Dr., 8006 Zürich (CH)
(74) Representative: Patentanwälte Ruff, Wilhelm, Beier, Dauster & Partner

(56) References cited:
- EP-A1- 1 543 848
- WO-A1-2006/084390
- US-A1- 2008 183 208

## Description

The invention relates to a textile implant having a sheath-core construction, to a tubular medical device and also to a manufacturing method for the textile implant and for the tubular medical device.

One of the challenges in modern surgical care is the surgical management of infected wounds and the avoidance of post-operative primary and secondary infections. Post-operative secondary infections are considered particularly problematic since they often only arise after several weeks or even months. This presents a particularly severe problem in the case of nonabsorbable implants in particular. In many cases, the implants have to be explanted again in order that the infections can be dealt with successfully. Such surgical interventions are the cause of generally higher costs in the hospital sector, lead to longer hospital stays and represent additional stress for the patients concerned.

This is why surgical implants, for example suture materials, hernia meshes, vascular prostheses but also knee and hip prostheses, are being increasingly endowed with suitable antimicrobial substances. An implant of this kind is described in DE 10 2004 047 568 A1 for example. One problem with antimicrobializing implants is generally that it is not rare for the antimicrobial substances to undergo an accumulated release into the surrounding biological tissue in the form of a brief and high-dosage "burst", and this may engender inflammatory and in some instances even necrotic changes in the tissue. A further disadvantage concerns particularly textile implants, for example suture materials, vascular prostheses, hernia meshes and the like. There, the presence of antimicrobial substances, particularly when the substances are incorporated in the implant as particles and exceed a certain particle size, can cause mechanical weakening of the implants. This weakening can manifest itself particularly in reduced mechanical strength, for example reduced linear breaking strength, knot breaking strength or flexural stiffness and, where appropriate, in a certain brittleness of the implant threads. Finally, the addition of antimicrobial substances to polymer materials results in many cases in worse processibility of the materials, which makes the manufacture operation for such suture materials or textile implants costly and inconvenient.

Recently there have also been disclosed fibre products where the fibre material has inherent antimicrobial, particularly antibacterial, properties. Such bioactives are known from WO 02/081791 A2 for example.High strength antimicrobial sutures with absorbable core and absorbable sheath are known from EP 1543848.

It is an object of the present invention to provide an antimicrobialized textile implant without eliciting the problems which are known from the prior art. The implant shall, more particularly, combine sufficient mechanical strength for surgical uses with sufficient antimicrobial efficacy. In addition, the implant shall be simple and, more particularly, inexpensive to manufacture.

It is a further object of the present invention to provide a medical device which similarly avoids the problems known from the prior art.

We have found that these objects are achieved according to the present invention by a textile implant having the features as per independent claims 1 and 2. Preferred embodiments of the textile implant are subject matter of dependent claims 3 to 17. The wording of all claims is hereby incorporated in this description by reference.

The implant according to the invention comprises a textile implant according to claims 1 and 2.

The invention provides a textile implant having a sheath-core construction and an antimicrobial additization. The antimicrobial additization, as already mentioned, is based on a composition comprising at least one antimicrobial active and silica as a support material for the at least one active. The composition contemplated according to the invention has the particular advantage of even in low concentrations ensuring a consistent and, more particularly, long-lasting delivery of the antimicrobial active. No accumulated, "burst effect" release of the active takes place, which distinctly reduces the risk of inflammatory or necrotic tissue changes.

The manner in which the polymeric sheath of the thread surrounds its polymeric core is generally complete and, more particularly, the entire area of the polymeric core is covered by the polymeric sheath. The sheath itself may have a construction featuring one, two, three or more layers. The proportion of the at least one thread which is attributable to the polymeric sheath is preferably between 10% and 60% by volume and particularly 20% and 50% by volume, based on the overall volume of the at least one thread.

Either only the polymeric core of the thread or only the polymeric sheath of the thread includes the composition contemplated according to the invention. This spatial separation, or compartmentization, into antimicrobially active and antimicrobially inactive regions makes it possible to reduce the proportion of the composition, in particular the proportion of the at least one antimicrobial active, in the at least one thread and hence the overall manufacturing costs for the implant according to the invention. This is of special economic interest particularly with regard to pricey actives, for example silver. It must be particularly emphasized that reducing the antimicrobial additization to the polymeric core or sheath of the thread does not compromise adequate antimicrobial protection.

In a particularly preferred embodiment, it is only the polymeric sheath which includes the composition; that is, the polymeric core of the thread is free of the composition. This advantageously preserves the mechanical strength/resistance of the core of the thread. The implant according to the invention can accordingly be used in surgery without reservations. Preferably, the composition is situated in the outermost layer of a polymeric sheath constructed in one, two, three or more layers. A further advantage of the embodiment described in this section is that the at least one antimicrobial active is present in surface-near regions of the at least one thread and hence in regions that are exposed to a particular risk of bacterial colonization.

In an alternative embodiment, it is only the polymeric core which includes the composition; that is, the polymeric sheath is free of the composition. Preferably, in this case, the polymeric core is formed of a nonabsorbable polymer material and the polymeric sheath of an absorbable polymer material.

The proportion of the polymeric core or of the polymeric sheath that is attributable to the composition is preferably between 50 and 100 000 ppm, particularly 50 and 40 000 ppm, more preferably 500 and 40 000 ppm, based on the overall weight of the polymeric core or of the polymeric sheath. The proportion of the polymeric core or of the polymeric sheath that is attributable to silica is between 40 and 95 000 ppm, particularly 400 and 38 000 ppm, based on the overall weight of the polymeric core or of the polymeric sheath. The proportion of the polymeric core or of the polymeric sheath that is attributable to the antimicrobial active is preferably between 10 and 5000 ppm, particularly 50 and 2000 ppm, more preferably 100 and 2000 ppm, particularly 100 and 1000 ppm, based on the overall weight of the polymeric core or of the polymeric sheath.

In a further developed embodiment, the proportion of the at least one thread that is attributable to the composition is between 5 and 60 000 ppm, particularly 50 and 24 000 ppm, preferably 250 and 10 000 ppm, particularly 500 and 5000 ppm, based on the overall weight of the at least one thread. The proportion of the at least one thread that is attributable to silica is preferably between 4 and 57 000 ppm, particularly 40 and 22 800 ppm, based on the overall weight of the at least one thread. The proportion of the at least one thread that is attributable to the antimicrobial active is preferably between 1 and 3000 ppm, particularly 10 and 1200 ppm, preferably 100 and 1000 ppm, based on the overall weight of the at least one thread.

It is further preferable according to the invention when the at least one thread includes the composition down to a depth (starting from the outside surface of the at least one thread) between 5 and 40%, particularly 5 and 30%, based on the overall thickness of the at least one thread.

The composition used in the present invention is preferably a powder and more particularly a fine and dusty powder. Depending on the antimicrobial active, the composition may have a colour or at least a tinge. The presence of silver nanoparticles, for example, may render the composition slightly yellowing to brownish.

The silica in the composition is preferably amorphous, in particular x-ray amorphous. The silica preferably has a three-dimensional and, more particularly, open structure, particularly in the manner of a matrix. The three-dimensional structure is preferably porous, in particular openly porous. The three-dimensional structure may have an interconnecting porosity, i.e. pores/voids which communicate via channels. In general, the at least one antimicrobial active is uniformly distributed in the silica, particularly in pores, channels and/or surfaces of the silica. The silica may have particles between 2 and 50 nm, particularly 5 and 30 nm and preferably 10 and 20 nm in diameter. The silica particles can also be present in the form of aggregates and/or agglomerates. Agglomerates may be organized into superordinate chain-like, in particular beaded chain-like, structures. The chain-like structures may in turn be combined into a superordinate structure featuring voids and connecting channels. The agglomerates themselves may have a diameter between 50 and 2000 nm, in particular 100 and 1000 nm. Agglomerates are usually rather loosely conjoined structures which can easily be rent asunder again under mechanical stress for example. This makes the composition particularly good for incorporation into a polymer material contemplated for the thread core and/or the sheath. In addition, the composition may also contain mixed agglomerates based on silicon and active-ingredient particles.

The composition preferably has a specific surface area between 100 and 400 m²/g. The proportion of the composition that is attributable to the at least one antimicrobial active is preferably between 1% and 50% by weight, particularly 5% and 50% by weight and more preferably 5% and 20% by weight, based on the overall weight of the composition. The proportion of the composition which is attributable to silicon dioxide is preferably between 80% and 99% by weight and particularly 80% and 95% by weight, based on the overall weight of the composition.

The at least one antimicrobial active preferably comprises antimicrobially active metals and/or metal compounds, in particular metal alloys and/or metal salts, particularly metal oxides. The at least one antimicrobial active is preferably a metal or a metal salt and, more particularly, selected from the group consisting of copper, silver, gold, zinc, titanium, copper oxide, silver oxide, zinc oxide and titanium dioxide. The use of silver and/or silver compounds, for example silver salts, is particularly preferred.

The at least one antimicrobial active may further comprise a mixture or a combination of a plurality of antimicrobial actives. Polyhexamethylenebiguanide and/or chlorhexidine and derivatives thereof are possible for example in addition to the actives heretofore described.

In a further embodiment, the at least one antimicrobial active is present in the form of particles. The at least one active may be present as nano- to microparticles, particularly in the form of nanoparticles. The particle diameter of the at least one antimicrobial active is preferably between 5 and 1000 nm, more preferably 5 and 600 nm, particularly 10 and 300 nm, even more preferably 5 and 20 nm. When the at least one antimicrobial active comprises metal particles, particularly metal nanoparticles, the antimicrobial composition contemplated according to the invention may be obtainable/obtained by following a flame spray pyrolysis process. A first step of this process generally comprises preparing a solution of a metal salt and a preferably volatile silicon compound in an organic solvent. Useful silicon compounds include, in particular, organic silanes, for example tetraethoxyorthosilane and/or hexamethyldisiloxane. Useful solvents include alcohols, particularly methanol, ethanol, n-propanol, n-butanol, isopropanol, ethanediol, propanediol and also mixtures thereof. In a second step, the solution is then sprayed into a flame having a temperature of about 1500°C. The flame is usually ignited by means of a gas mixture, for example of methane and oxygen. Thereafter, the flame generally maintains itself by burning the solution.

With regard to further features and details of the heretofore described composition and also concerning the flame spray pyrolysis process described in the preceding section, reference is made to WO 2006/084411 A1 and WO 2006/084390 A1.

The implant according to the invention is particularly advantageous in offering long-term antimicrobial protection. The antimicrobial protection offered by the implant preferably extends for a period ranging from several months to several years. In the case of a permanent implant, antimicrobial protection can extend to a period of 10 to 15 years.

The at least one thread in a further embodiment has a linear density between 1 and 3500 dtex, particularly 20 and 1000 dtex, preferably 150 and 250 dtex. The customarily used thread gauges are contemplated, particularly when the at least one thread is configured as surgical suture, in particular USP-8/0, USP-7/0, USP-6/0, USP-5/0, USP-4/0, USP-3/0, USP-2/0, USP-0, USP-1, USP-2, USP-3, USP-4, USP-5 and/or USP-6, preferably USP-8/0, USP-7/0, USP-6/0, USP-5/0, USP-4/0, USP-3/0, USP-2/0, USP-0, USP-1 and/or USP-2.

The linear tenacity (tensile strength) of the at least one thread is preferably between 10 and 100 cN/tex, particularly 40 and 80 cN/tex. When the implant according to the invention is a surgical suture, then the at least one thread preferably has a linear tenacity between 20 and 60 cN/tex. When, by contrast, the implant according to the invention is a textile mesh, then mesh threads can have a linear tenacity between 30 and 100 cN/tex, particularly 40 and 80 cN/tex. Linear tenacity herein is to be understood as meaning the force which is measured in tensile tests and force and distance are recorded. The at least one thread may further have a diameter between 0.005 and 1 mm, particularly 0.05 and 0.5 mm. The diameter of the thread core may according to the invention be between 40 and 95% of the overall diameter, i.e. the diameter of the at least one thread. The diameter of the polymeric core is preferably between 8 and 750 µm, particularly 12 and 600 µm.

The at least one thread preferably comprises a monofilament. However, the at least one thread may also be present as a multifilament, in particular a multifilament yarn, in which case individual threads, preferably all individual threads, of the multifilament have the sheath-core structure according to the invention.

The polymeric core is formed of nonabsorbable polymer materials. The polymeric sheath may in principle be formed of an absorbable or nonabsorbable polymer material. The polymer material may also be present as homo-, co-, tri- or tetrapolymer etc. The material may in particular be present as block copolymer or block terpolymer.

Useful nonabsorbable polymer materials include particularly polyolefins, polyesters, fluoropolymers, polyamides, polyurethanes and/or copolymers thereof. The nonabsorbable polymer material is preferably selected from at least one material from the group consisting of polyethylene, polypropylene, polytetrafluoroethylene, polyethylene terephthalate, polytetrafluoroethylene, polyvinylidene difluoride and copolymers of vinylidene difluoride and hexafluoropropylene. Polytetrafluoroethylene preferably comprises expanded polytetrafluoroethylene.

Useful absorbable polymer materials include particularly polymers, especially co- or terpolymers, based on hydroxy carboxylic acid units. The absorbable polymer material is preferably at least one polymer from the group consisting of polylactide, polyglycolide, poly(trimethylene carbonate), poly(ε-caprolactone), poly(para-dioxanone), poly(hydroxybutyric acid) and copolymers thereof.

In a further developed embodiment, the polymeric core and the polymeric sheath are formed of the same polymer material.

In an alternative embodiment, the polymeric core and the polymeric sheath are formed of different polymer materials. More particularly, the polymeric core may be formed of a nonabsorbable polymer material and the polymeric sheath of an absorbable polymer material. As mentioned earlier, it is in this case when it is only the polymeric core which includes the composition. In a further developed embodiment, the polymeric core is formed of a nonabsorbable polymer material and the polymeric sheath of an absorbable polymer material, in which case the polymeric core includes at least one antimicrobial active having a long-term effect, for example silver, and the polymeric sheath includes at least one antimicrobial active having a short-term effect, for example polyhexamethylenebiguanide and/or chlorhexidine, particularly in the form of a surficial coating.

In further embodiments, the at least one thread includes not only the composition contemplated according to the invention but also additional additives. These additives may comprise for example binding or bonding agents, growth factors, analgesics, anti-inflammatories and/or x-ray contrast media, particularly barium sulphate.

In a preferred embodiment, the at least one thread comprises a plurality of threads, particularly a multiplicity of threads, preferably in the form of a multifilament. These threads are generally present in a form which has been subjected to textile processing. The implant preferably comprises a textile fabric, preferably a woven fabric, a (loop-formingly) knitted fabric, a (loop-drawingly) knitted fabric, a braided fabric, a nonwoven scrim or a nonwoven fabric.

In a particularly preferred embodiment, the implant comprises a textile mesh, preferably a loop-formingly knitted textile mesh. The mesh may have openings having an open width between 0.05 and 8 mm, in particular 0.1 and 6 mm. The basis weight of the mesh is preferably between 25 and 100 g/cm², particularly 30 and 80 g/cm². In a further developed embodiment, the textile mesh elongates by 90 to 230% when subjected to a maximum tensile force Fₘₐₓ of 10 to 100 N/cm across the knitted direction of the mesh and by 50 to 250% when subjected to a maximum tensile load Fₘₐₓ of 10 to 100 N/cm along the knitted direction of the mesh.

The implant preferably comprises a mesh selected from the group consisting of hernia mesh, hernia plug, prolapse mesh and urine incontinence mesh. According to the invention, however, the textile implant may also comprise an endoprosthesis, particularly a stent or vascular prosthesis, or may comprise a textile band.

In a further preferred embodiment, the implant comprises a surgical suture material.

The present invention further provides a tubular medical device having an at least two-layered construction comprising an inner layer surrounding a tubular lumen and an outer layer surrounding the inner layer, wherein the inner layer or the outer layer include a composition comprising at least one silica-supported antimicrobial active. It is only the inner layer or only the outer layer which includes the composition. This compartmentization of the composition, particularly of the at least one silica-supported antimicrobial active, makes it possible for the advantages mentioned above in connection with the textile implant to be also actualized in relation to the tubular device described in this section.

In a further developed embodiment, the tubular medical device has a three-layered construction. Preferably, the device has an interlayer between the inner and outer layers. In this embodiment, the device thus includes an inner layer surrounding a tubular lumen, an interlayer surrounding the inner layer and an outer layer surrounding the interlayer. Preferably, it is only the inner and outer layers which include the composition contemplated according to the invention. As a result, it is only the accessible surfaces (the inner and outer layers) which are rendered antimicrobial. The interlayer, by contrast, is preferably free of the composition, leaving particularly the mechanical properties of the interlayer intact.

The medical device preferably comprises a tubular extrudate. For example, the device may be configured as a catheter, particularly a bladder, dialysis, heart, nephrostomy, peridural, port, tube, urethral or venous catheter. It can similarly be possible, according to the invention, for the medical device to comprise a trocar, drainage tube or irrigation tube.

With regard to further features and details, particularly in relation to the composition, reference is made to the description heretofore.
The present invention also provides a method of forming the textile implant, wherein a molten thread core polymer and a molten sheathing polymer are coextruded to form sheathed threads, i.e. threads having a sheath-core construction, and the sheathed threads are subsequently processed into a textile implant, wherein the composition is incorporated into the thread core polymer or into the sheathing polymer, only the thread core polymer or only the sheathing polymer and more preferably only the sheathing polymer.

In general, the sheathing polymer is coextruded onto the outer circumference of the thread core polymer as long as the thread core polymer is still soft.

In a suitable embodiment, the composition is incorporated into the thread core polymer or into the sheathing polymer before the coextrusion step. Preferably, the thread core polymer and/or the sheathing polymer is compounded with the composition. Compounding preferably uses a masterbatch. A masterbatch comprises a concentrate of the composition contemplated according to the invention in the thread core polymer and/or sheathing polymer. To form the masterbatch, the composition is usually mixed with the thread core or sheathing polymer, melted and subsequently extruded. The final shaping of the masterbatch can be effected by pelletizing the extruded material. For instance, the extruded material can be further processed into pellets by strand pelletization or underwater pelletization for example. The masterbatch can subsequently be remelted and used together with a melt of the thread core polymer and/or a melt of the sheathing polymer for coextrusion to form the thread according to the invention.

The proportion of the melts used for coextrusion that is attributable to the composition can be between 50 and 100 000 ppm, particularly 50 and 40 000 ppm, based on the overall weight of the melts. The proportion of the melts that is attributable to the antimicrobial active can be between 10 and 5000 ppm, particularly 100 and 2000 ppm, based on the overall weight of the melts. The melts mentioned in this section preferably comprise the melt of the sheathing polymer.

It is in principle also possible to incorporate the composition into the thread core or sheathing polymer even as it is being synthesized.

In principle all suitable textile techniques are contemplated for processing the coextruded sheath-core threads into a textile implant. These suitable textile techniques are sufficiently well known to a person skilled in the art, so that a detailed description is dispensed with here.

The present invention finally also provides a method of forming the tubular medical device, wherein at least two polymer melts are coextruded to form an at least two-layered tubular medical device having an inner layer surrounding a tubular lumen and an outer layer surrounding the inner layer and a composition comprising at least one silica-supported antimicrobial active is incorporated into only one of the polymer melts. To form a three-layered device, three polymer melts are generally used. It is in this case when the composition contemplated according to the invention is incorporated into two of the polymer melts, preferably into the polymer melts for the inner and outer layers. With regard to further features and details, reference is made as far as possible to the description heretofore.

Further features of the invention will be apparent from the subsequent description of preferred embodiments with reference to examples in conjunction with the features of the subclaims. The individual features of the invention can be actualized therein alone or in combination with one another.

### Example 1:

### Preparation of a masterbatch of silver, silica and polypropylene

A twin-screw extruder was charged with about 10 kg of medical-grade polypropylene. The polypropylene was melted at a temperature of about 180°C. Then, about 300 g of a composition of amorphous silica and silver nanoparticles were admixed to the molten polypropylene, so that the proportion of the mixture that was attributable to the composition was about 3% by weight. Thereafter, the molten mixture was extruded. This material was pelletized after cooling and used as a masterbatch having a silver content of about 5000 ppm.

### Example 2:

### Preparation of antimicrobially active polyethylene terephthalate fibres having a sheath-core construction

Coextrusion was used to produce polyethylene terephthalate fibres having a sheath-core construction and including in the sheath an antimicrobial composition comprising silicon dioxide as support material and silver as antimicrobial active. Then, the fibres were subjected to antimicrobiological tests corresponding to the requirements of the Japanese industrial standard governing the antibacterial activity and efficacy of textile products (JIS L 1902). To this end, 0.4 g of a fibre sample was wound into dense bundles in 3-plicate. The bundles were sterilized with 70 per cent ethanol and then dried. Each filament bundle was subsequently inoculated with 50 µl of a bacterial culture containing about 3 * 10⁵ colony-forming units of Klebsiella pneumoniae (DSM 789) per cm³, and incubated at 37°C for 18 hours. Thereafter, still viable bacteria were rinsed off every filament bundle, multiplied and counted. The results found are listed in Table 1 below.

**Table 1**

| # | Type | Core | Sheath | ppm Ag | Average value of colony-forming units per cm³ | Reduction in [%] |
|---|---|---|---|---|---|---|
| 1 | monofilament control (0 h) | - | - | 0 | 3.3 × 10⁵ | - |
| 1 | monofilament (control) | - | - | 0 | 3.8 × 10⁵ | - |
| 2 | monofilament | Ag | Ag | 100 | 1.1 × 10⁵ | 72.35% |
| 3 | monofilament | Ag | Ag | 500 | 1.5 × 10³ | 99.60% |
| 4 | silver in sheath | - | Ag | 500 | 9.9 × 10¹ | 99.97% |
| 5 | silver in core | Ag | - | 500 | 2.9 × 10⁵ | 25.57% |

## Claims

1. A textile implant comprising at least one thread having a polymeric core and a polymeric sheath which surrounds the polymeric core at least partly, wherein only the polymeric sheath includes a composition comprising at least one silica-supported antimicrobial active and wherein the polymeric core and the polymeric sheath are formed of a nonabsorbable polymer material.

2. A texile implant comprising at least one thread having a polymeric core and a polymeric sheath which at least partly surrounds the polymeric core, wherein only the polymeric core includes a composition comprising at least one silica-supported antimicrobial active agent wherein the polymeric core is formed of a nonabsorbable polymer material and the polymeric sheath is formed of an absorbable polymer material.

3. A textile implant according to claim 1 or 2, **characterized in that** the entire area of the polymeric core is covered by the polymeric sheath.

4. A textile implant according to any preceeding claim, **characterized in that** the proportion of the at least one thread which is accounted for by the polymeric sheath is between 10% and 60% by volume, in particular 20% and 50% by volume.

5. A textile implant according to claim 1, **characterized in that** the composition is situated in the outermost layer of a sheath constructed in one, two, three or more layers.

6. A textile implant according to any preceding claim, **characterized in that** the proportion of the polymeric core or of the polymeric sheath that is attributable to the composition is between 50 and 100 000 ppm.

7. A textile implant according to any preceding claim, **characterized in that** the proportion of the polymeric core or of the polymeric sheath that is attributable to the at least one antimicrobial active is between 10 and 5000 ppm.

8. A textile implant according to any preceding claim, **characterized in that** the at least one antimicrobial active is a metal and/or a metal compound, preferably metal salt, particularly from the group consisting of copper, silver, gold, zinc, titanium, copper oxide, silver oxide, zinc oxide and titanium dioxide, wherein preferably the at least one antimicrobial active comprises silver and/or silver compounds, particularly silver salts.

9. A textile implant according to any preceding claim, **characterized in that** the at least one antimicrobial active is present in the form of particles, particularly nanoparticles, wherein preferably the at least one antimicrobial active has a particle diameter between 5 and 1000 nm, particularly 10 and 300 nm.

10. A textile implant according to any preceding claim, **characterized in that** the at least one thread has a linear tensile strenght between 10 and 100 cN/tex, particularly 40 and 80 cN/tex.

11. A textile implant according to claim 1, **characterized in that** the polymeric core and the polymeric sheath are formed of the same polymer material.

12. A textile implant according to any one of claims 1 to 10, **characterized in that** the polymeric core and the polymeric sheath are formed of different polymer materials.

13. A textile implant according to any preceding claim, **characterized in that** the at least one thread comprises a plurality of threads, more particularly a multiplicity of threads, preferably in the form of a multifilament.

14. A textile implant according to any preceding claim, **characterized in that** the implant comprises a textile fabric, particularly a woven fabric, a knitted fabric, a braided fabric or a nonwoven fabric.

15. A textile implant according to any preceding claim, **characterized in that** the implant comprises a textile mesh, preferably from the group consisting of hernia mesh, hernia plug, prolapse mesh and urine incontinence mesh.

16. A textile implant according to any one of claims 1 to 14, **characterized in that** the implant is an endoprosthesis, particularly a vascular prosthesis.

17. A textile implant according to any one of claims 1 to 14, **characterized in that** the implant is a surgical suture material.

## Patentansprüche

1. Textiles Implantat, umfassend zumindest einen Faden mit einem polymeren Kern und einer den polymeren Kern zumindest teilweise umgebenden polymeren Ummantelung, wobei nur die polymere Ummantelung eine Zusammensetzung, umfassend zumindest einen Siliciumoxid geträgerten antimikrobiellen Wirkstoff, aufweist, und wobei der polymere Kern und die polymere Ummantelung aus einem nichtresorbierbaren Polymermaterial gebildet sind.

2. Textiles Implantat, umfassend zumindest einen Faden mit einem polymeren Kern und einer den polymeren Kern zumindest teilweise umgebenden polymeren Ummantelung, wobei nur der polymere Kern eine Zusammensetzung, umfassend zumindest einen Siliciumoxid geträgerten antimikrobiellen Wirkstoff, aufweist, wobei der polymere Kern aus einem nichtresorbierbaren Polymermaterial gebildet ist und die polymere Ummantelung aus einem resorbierbaren Polymermaterial gebildet ist.

3. Textiles Implantat nach Anspruch 1 oder 2, **dadurch gekennzeichnet, dass** der gesamte Bereich des polymeren Kerns von der polymeren Ummantelung bedeckt ist.

4. Textiles Implantat nach einem der vorhergehenden Ansprüche, **dadurch gekennzeichnet, dass** der Anteil der polymeren Ummantelung in dem zumindest einen Faden zwischen 10 Volumen-% und 60 Volumen-%, insbesondere 20 Volumen-% und 50 Volumen-% beträgt.

5. Textiles Implantat nach Anspruch 1, **dadurch gekennzeichnet, dass** sich die Zusammensetzung in der äußersten Schicht einer ein-, zwei-, drei- oder mehrschichtig aufgebauten Ummantelung befindet.

6. Textiles Implantat nach einem der vorhergehenden Ansprüche, **dadurch gekennzeichnet, dass** die Zusammensetzung in dem polymeren Kern oder in der polymeren Ummantelung einen Anteil zwischen 50 und 100 000 ppm aufweist.

7. Textiles Implantat nach einem der vorhergehenden Ansprüche, **dadurch gekennzeichnet, dass** der zumindest eine antimikrobielle Wirkstoff in dem polymeren Kern oder der polymeren Ummantelung einen Anteil zwischen 10 und 5000 ppm aufweist.

8. Textiles Implantat nach einem der vorhergehenden Ansprüche, **dadurch gekennzeichnet, dass** der zumindest eine antimikrobielle Wirkstoff ein Metall und/oder eine Metallverbindung, bevorzugt ein Metallsalz, ist, insbesondere aus der Gruppe bestehend aus Kupfer, Silber, Gold, Zink, Titan, Kupferoxid, Silberoxid, Zinkoxid und Titanoxid, wobei vorzugsweise der zumindest eine antimikrobielle Wirkstoff Silber und/oder Silberverbindungen, insbesondere Silbersalze, umfasst.

9. Textiles Implantat nach einem der vorhergehenden Ansprüche, **dadurch gekennzeichnet, dass** der zumindest eine antimikrobielle Wirkstoff in Form von Partikeln, insbesondere Nanopartikeln, vorliegt, wobei vorzugsweise der zumindest eine antimikrobielle Wirkstoff einen Partikeldurchmesser zwischen 5 und 1000 nm, insbesondere 10 und 300 nm, aufweist.

10. Textiles Implantat nach einem der vorhergehenden Ansprüche, **dadurch gekennzeichnet, dass** der zumindest eine Faden eine lineare Zugfestigkeit zwischen 10 und 100 cN/tex, insbesondere 40 und 80 cN/tex, aufweist.

11. Textiles Implantat nach Anspruch 1, **dadurch gekennzeichnet, dass** der polymere Kern und die polymere Ummantelung aus dem gleichen Polymermaterial gebildet sind.

12. Textiles Implantat nach einem der Ansprüche 1 bis 10, **dadurch gekennzeichnet, dass** der polymere Kern und die polymere Ummantelung aus verschiedenen Polymermaterialien gebildet sind.

13. Textiles Implantat nach einem der vorhergehenden Ansprüche, **dadurch gekennzeichnet, dass** der zumindest eine Faden eine Mehrzahl von Fäden, insbesondere eine Vielzahl von Fäden, vorzugsweise in Form eines Multifilaments, umfasst.

14. Textiles Implantat nach einem der vorhergehenden Ansprüche, **dadurch gekennzeichnet, dass** es sich bei dem Implantat um ein textiles Flächengebilde, insbesondere ein Gewebe, Gewirke, Geflecht oder Vlies, handelt.

15. Textiles Implantat nach einem der vorhergehenden Ansprüche, **dadurch gekennzeichnet, dass** es sich bei dem Implantat um ein textiles Netz, vorzugsweise aus der Gruppe bestehend aus Herniennetz, Hernienplug, Prolapsnetz und Harninkontinenznetz, handelt.

16. Textiles Implantat nach einem der Ansprüche 1 bis 14, **dadurch gekennzeichnet, dass** das Implantat als Endoprothese, insbesondere Gefäßprothese, ausgebildet ist.

17. Textiles Implantat nach einem der Ansprüche 1 bis 14, **dadurch gekennzeichnet, dass** das Implantat als chirurgisches Nahtmaterial ausgebildet ist.

## Revendications

1. Implant textile comprenant au moins un fil présentant un noyau polymère et une gaine polymère qui entoure au moins partiellement le noyau polymère, dans lequel seule la gaine polymère comprend une composition comprenant au moins un agent actif antimicrobien à support de silice, dans lequel le noyau polymère et la gaine polymère sont constitués d'une matière polymère non absorbable.

2. Implant textile comprenant au moins un fil présentant un noyau polymère et une gaine polymère qui entoure au moins partiellement le noyau polymère, dans lequel seul le noyau polymère comprend une composition comprenant au moins un agent actif antimicrobien à support de silice, dans lequel le noyau polymère est constitué d'une matière polymère non absorbable, et la gaine polymère est constituée d'une matière polymère absorbable.

3. Implant textile selon la revendication 1 ou 2, **caractérisé en ce que** la totalité de la surface du noyau polymère est recouverte par la gaine polymère.

4. Implant textile selon l'une quelconque des revendications précédentes, **caractérisé en ce que** la proportion dudit au moins un fil qui est utilisée par la gaine polymère est comprise entre 10 % en volume et 60 % en volume, en particulier entre 20 % en volume et 50 % en volume.

5. Implant textile selon la revendication 1, **caractérisé en ce que** la composition est située dans la couche extérieure extrême d'une gaine qui est constituée d'une couche, ou de deux, trois ou plus de trois couches.

6. Implant textile selon l'une quelconque des revendications précédentes, **caractérisé en ce que** la proportion du noyau polymère ou de la gaine polymère qui est attribuable à la composition est comprise entre 50 ppm et 100000 ppm.

7. Implant textile selon l'une quelconque des revendications précédentes, **caractérisé en ce que** la proportion du noyau polymère ou de la gaine polymère qui est attribuable audit au moins un agent actif antimicrobien est comprise entre 10 ppm et 5000 ppm.

8. Implant textile selon l'une quelconque des revendications précédentes, **caractérisé en ce que** ledit au moins un agent actif antimicrobien est un métal et/ou un composé de métal, de préférence un sel de métal, en particulier issu du groupe comprenant le cuivre, l'argent, l'or, le zinc, le titane, l'oxyde de cuivre, l'oxyde d'argent, l'oxyde de zinc et le dioxyde de titane, dans lequel ledit au moins un agent actif antimicrobien contient de préférence de l'argent et/ou des composés d'argent, en particulier des sels d'argent.

9. Implant textile selon l'une quelconque des revendications précédentes, **caractérisé en ce que** ledit au moins un agent actif antimicrobien est présent sous la forme de particules, en particulier de nanoparticules, dans lequel ledit au moins un agent actif antimicrobien présente de préférence un diamètre de particule qui est compris entre 5 nm et 1000 nm, en particulier entre 10 nm et 300 nm.

10. Implant textile selon l'une quelconque des revendications précédentes, **caractérisé en ce que** ledit au moins un fil présente une résistance à la traction linéaire qui est comprise entre 10 cN/tex et 100 cN/tex, en particulier entre 40 cN/tex et 80 cN/tex.

11. Implant textile selon la revendication 1, **caractérisé en ce que** le noyau polymère et la gaine polymère sont constitués de la même matière polymère.

12. Implant textile selon l'une quelconque des revendications 1 à 10, **caractérisé en ce que** le noyau polymère et la gaine polymère sont constitués de matières polymères différentes.

13. Implant textile selon l'une quelconque des revendications précédentes, **caractérisé en ce que** ledit au moins un fil comprend une pluralité de fils, plus particulièrement une multiplicité de fils, de préférence sous la forme d'un multifilament.

14. Implant textile selon l'une quelconque des revendications précédentes, **caractérisé en ce que** l'implant comprend un tissu textile, en particulier un tissu tissé, un tissu tricoté, un tissu tressé ou un tissu non tissé.

15. Implant textile selon l'une quelconque des revendications précédentes, **caractérisé en ce que** l'implant comprend un maillage textile, de préférence appartenant au groupe comprenant un filet anti-hernie, un bouchon anti-hernie, un filet anti-prolapsus et un filet anti-incontinence urinaire.

16. Implant textile selon l'une quelconque des revendications 1 à 14, **caractérisé en ce que** l'implant est une endoprothèse, en particulier une endoprothèse vasculaire.

17. Implant textile selon l'une quelconque des revendications 1 à 14, **caractérisé en ce que** l'implant est une matière de suture chirurgicale.
